# EUROPEAN PATENT APPLICATION

(11) **EP 1 985 300 A2**
(43) Date of publication of application: **29.10.2008**
(21) Application number: 07021976.1
(22) Date of filing: 13.11.2007
(51) Int. Cl.: A61K 36/06, A61K 36/07

(54) **A compound for re-equilibrating the intestinal bacterial flora with an antidiarrhoeic, anti-inflammatory and lentive activity against lactose and lactose derivative intolerances**

(30) Priority: 30.03.2007 IT MI20070649
(71) Applicant: Farmaceutici S.R.L., 15057 Tortona, Alessandria (IT)
(72) Inventor: Bonabello, Elvio, 15057 Tortona (Alessandria) (IT)
(74) Representative: Cicogna, Franco

(57) **Abstract**

A compound for a compound for re-equilibrating the intestinal bacterial flora with an anti-diarrhoeic, anti-inflammatory and lenitive activity against lactose and lactose derivative intolerances, characterized in that said compound comprises a compound base including Lactobacillus Sporogenes bacteric cells.

## Description

### BACKGROUND OF THE INVENTION

The present invention relates to a compound for re-equilibrating the intestinal bacterial flora with an anti-diarrhoeic, anti-inflammatory and lenitive activity against lactose and lactose derivative intolerances.

As is known, in the last twenty years, the problem of the intestinal dismicrobism has been addressed by different compounds including bacteric cells able of colonizing human intestinal mucose and, accordingly, hindering an uncontrolled growth of bacteria having a potentially pathogenic activity.

The first example of the above mentioned first generation compounds has been Enterogermine, based on the Bacillus Subtilis strain.

The Bacillus Subtilis strain is a sporogenic bacterium having the great advantage that it can be industrially processed without any loss of cellular vitality and that it does not require any storing and shipment precautions.

However, the above mentioned compound has a main limitation that the bacterium acts, against pathogenic agents, only by a "territorial competition", but it does not have any other useful activity for the host organism.

This limitation has compelled the searchers to perform a plurality of laboratory searches, to provide a patient with improved advantages, which searches have brought to the formulation of second generation products, the so-called lactic ferments.

The latter comprise suitably selected cells, capable of colonizing human intestinal mucose and being characterized in that they are also capable of producing useful substances for an enhanced human wellbeing.

Of the above mentioned substances, the most important from a physiologic standpoint comprise enzymes adapted to split lactose, as well as those substances deconjugating biliar acids, essential aminoacids and vitarilins.

However, since the above mentioned cells are not able to produce spores, they have a very small survival capability.

In order to overcome the above drawback, an early cellular lyophilization system has been applied, consisting of freezing and then dehydrating bacteria, before performing their production, storing and shipment procedures.

However, the above method is affected by a very great problem: in fact, the compounds must be preserved in a refrigerator at a temperature of 4°C, and the cold chain must be also maintained during the shipment, since, if not, the lactic bacteria would be subjected to a cellular death.

Subsequently, third-generation lactic ferments have been designed, by using the so-called "dry matrix dry spray" lyophilization system, which lactic ferments are still commercially available, and are adapted for re-equilibrating the intestinal bacteric flora.

The selected bacteric strains were the same already previously used, but they have been dehydrated by a forced ventilation system, to a moisture rate as near as possible to zero, thereby eliminating any water molecules, in the form of ice, at the center of the cells, and consequently removing the requirement of preserving in a refrigerator the finished product.

However, the above mentioned system is very aggressive for the lactic bacteria, and the loss of vital cells can also amount to 90%.

The above has compelled to use several billions of lactic ferments for each daily administering, thereby the above products are scarcely convenient.

Moreover, it has been also found that several surviving bacteria loose, because of the cytoplasmatic or nuclear modifications caused by the lyophilization, their replication capability, thereby their activity is only a temporary one.

### SUMMARY OF THE INVENTION

Accordingly, the aim of the present invention is to provide a compound adapted to solve the above mentioned problems.

Within the scope of the above aim, a main object of the invention is to provide such a compound which has a high intestinal bacteric flora re-equilibrating activity, is adapted to hinder the growth and development of pathogenic intestinal bacteria causing persisting diarrhoeic conditions, adapted to hinder or resist against dismicrobisms due to an antibiotic therapy, reduce symptoms in a case of an intolerance to lactose and derivatives thereof, provide a pro-biotic activity with respect to the host organism, and adapted to efficiently hinder vaginites and vulvo-vaginites caused by a cologenital bacteric translocation in women, sensitive to such a pathology type.

A further important object of the invention is to provide such a compound for re-equilibrating intestinal microflora which is not affected by a cellular death deriving from industrial processes, does not require storing or shipment at a controlled temperature, for example of 4°C, and which, as administered to a user, is not subjected to gastric transport losses, due to an acid PH and which, moreover, upon arriving at the patient duodenum is adapted to provide its pro-biotic functions for the host organism, such as:
- a production of Beta-galactosidasis enzymes, allowing to use lactose to produce lactic acid and adapted to make lactose digestible even for an intolerant person,
- a production of hydrolysis enzymes, adapted to deconjugate biliar acids,
- a production of antibiotic-like substances (bacteriocins and proteic complex compounds having a bacteriocidal activity),
- a production of the hydroxy-methyl-glutarate enzyme, COA, preventing the synthesis of cholesterol,
- a competitive inhibition of the pathogenic bacteria, and
- a provision of an acid environment.

According to one aspect of the present invention, the above mentioned aim and objects, as well as yet other objects, which will become more apparent hereinafter, are achieved by a compound for re-equilibrating intestinal dismicriobism, characterized in that said compound comprises a compound base consisting of the bacteric cells Lactobacillus Sporogenes.

Further characteristics and advantages of the present invention will become more apparent hereinafter from the following detailed disclosure of a compound for human intestinal mucose, having a re-equilibrating and protective activity, which will be specifically disclosed with reference to a specific embodiment example, for a better understanding of the invention.

In this connection it should be apparent that the inventive compound would be susceptible to several composition variations, all of which will come within the scope of the invention.

As is known, Lactobacillus Sporogenes has been for the first time insulated in 1933 by two American microbiologists which classified it under the name "Bacillus Coagulans".

Only few years ago (1997), two Japanese searchers have discovered that this sporogenic bacterium has all the methabolism characteristics of lactic ferments, thereby providing a novel formulation capability with respect to the up to now commercially available products.

Actually, the "Bergey's Manual of Determinative Bacteriology", in its seventh and last edition, has re-classified the Bacillus Coagulans under the name "Lactobacillus Sporogenes".

The sporogenic bacteria, as they are removed from their vital environment, instead of being subjected to a cellular death, produce a "capsule" or spore, therewithin they are able of surviving, even in very stringent conditions, while remaining at a quiescent status.

Then, if they are brought to a suitable environment, they will return to a germinating status, and will start to growth and replicate, thereby colonizing such an environment.

Laboratory tests performed on the above bacterical strain have shown a pro-biotic type of activity, indicative both of a human level use and of a veterinary level use.

The term "probiotic", for a lactic ferment, means a series of peculiar characteristics as acquired by the most recent scientific search: in fact, the lactic bacteria, to provide an important function in the intestinal euadjustment, must meet the following requirements:
- they must be of a metabolic nature "i.e. adapted to produce lactic acid and antibacteric substances, to deconjugate the biliar acids, degradate the nitrosamines and anticariogenic activity",
- they must be able of properly adhering to intestinal epithelia,
- they must be adapted to properly settle and provide an intestinal colonization,
- they must be adapted to reduce the blood cholesterol absorption, and
- they must be adapted to enhance the immune defenses.

Laboratory tests, performed both in vivo and in vitro, have shown that Lactobacillus Sporogenes, perfectly meets all the above mentioned requirements.

This component of the compound according to the present invention is used in an amount varying from a hundred million and a hundred billion of vital cells.

Moreover, to the subject compound another vital cell type is added, that is Streptococcus Termophilus, having the feature of a good survival to lyophilization, together with an optimum resistance against the gastric acidity.

The latter provide the inventive compound with a very good efficiency in processing against Helicobacter Pilorii eradication.

The latter component of the compound according to the present invention is applied in an amount varying from a hundred million of lyophilized life cells to a hundred billion thereof.

For a quicker and more intensive activity of the compound according to the present invention, lyophilized cells of Saccaromices Cerevisiae have been further added.

The latter yeasts, even if they do not come within the lactic ferment range, and even if they do not colonize in a stable manner human intestinal mucose, are characterized by very intensive metabolic activities, with a good production of aminoacids, enzymes and vitamins, forming a substrate which is very rich in nourishing substances, adapted to enhance the growth and replication rate of Lactobacillus Sporogenes and Streptococchi Termophili.

Thus, a compound is herein provided having a very enhanced efficiency, both from the activity rate standpoint, and from an effect duration standpoint.

This component of the compound according to the present invention is introduced in an amount from one to fifty per cent of the overall weight of the daily administration dose.

Since the compound according to the present invention is very useful for preventing diarrhoea caused by an antibiotic-cotherapy, and since a use of antibiotics causes a loss of the vitaminic reserves, the following vitamins are moreover introduced in the compound, to further improve the product:
- Vitamin B1
- Vitamin B2
- Vitamin B6
- Vitamin PP

The provided amount for each vitamin can be varied from a minimum of fifty percent of the rational daily dose to twice said daily dose.

No vitamin B12 has been deliberately used, differently from all the other makers of like products, since said vitamin is a cellular growth stimulating vitamin and, accordingly, it could modulate an increase of possible cancerogenic masses in patients affected by a tumoral neoplasia.

In fact, since the above patients, subjected to chemiotherapy and radiating therapy, are affected by a very strong intestinal dismicrobism, it has been found as very suitable to formulate a compound also useful for these patients.

Moreover, since the main problem is that of the probiotic cellular material loss, which is moreover the most important problem related to a formulation of an efficient lactic ferment, the laboratory results has been further subjected to a stability test.

To perform such a control procedure, the test in the "zone 4" has been selected.

Actually, the stability test performed in "zone 4" represents an accelerated simulation of the most stringent conditions, the stability of a pharmaceutical composition can be subjected to.

Such a condition has been identified at a constant temperature of 40°C and with a moisture rate of 75%.

From the above disclosure it should be apparent that the invention fully achieves the intended aim and objects.

In fact, the invention provides a compound based on the use of a particular type of cells which, since they are adapted to produce spores, are held in a vital and survival condition up to their expiry date, and accordingly up to the moment thereat they are administered to the patient, independently from the manner to which the product has been preserved.

Those same cells, moreover, are adapted to pass through the acid medium of the gastric apparatus, without being subjected to either qualitative or quantitative modifications, thereby providing an efficient and stable colonization of human intestinal mucose.

The compound, as disclosed, is susceptible to several modifications and variations, all of which will come within the scope of the invention.

Moreover, all the component substances of the subject compound can be replaced by other substances having equivalent characteristics.

## Claims

1. A compound for re-equilibrating the intestinal bacteria flora with an anti-diarrhoeic, anti-inflammatory and lenitive activity against lactose and lactose derivative intolerances, **characterized in that** said compound comprises a compound base consisting of Lactobacillus Sporogenes.

2. A compound, according to claim 1, **characterized in that** said Lactobacillus Sporogenes is present in said compound in an amount varying from a hundred million and a hundred billion of vital cells.

3. A compound, according to claim 1 or 2, **characterized in that** said compound further comprises Streptococcus Termophilus.

4. A compound, according to claim 3, **characterized in that** said Streptococcus Termophilus is included in said compound in an amount varying from a hundred million and a hundred billion of lyophilized vital cells.

5. A compound, according to one or more of the preceding claims, **characterized in that** said compound further comprises Saccaromices Cerevisiae lyophilized cells.

6. A compound, according to one or more of the preceding claims, **characterized in that** said Saccaromices Cerevisiae lyophilized cells are present in said compound in an amount from 1 to 50% of an overall weight of a daily administering dose of said compound.

7. A compound, according to one or more of the preceding claims, **characterized in that** said compound further comprises vitamins B1, B2, B6 and PP.

8. A compound, according to one or more of the preceding claims, **characterized in that** each said vitamin is present in said compound in an amount from a minimum amount of 50% of a daily administering dose, to a twice amount of said minimum amount.

9. A compound, according to one or more of the preceding claims, **characterized in that** said compound comprises one or more of the disclosed and/or illustrated characteristics.
